# EUROPEAN PATENT SPECIFICATION

(11) **EP 0 345 974 B1**
(45) Date of publication and mention of the grant of the patent: **29.12.1993**
(21) Application number: 89305327.2
(22) Date of filing: 25.05.1989
(51) Int. Cl.: A61K 37/02, C12N 5/00, C07K 15/06

(54) **Cell-proliferative proteins and isolation thereof**
Auf das Zellwachstum wirkende Proteine und ihre isolierung
Protéines à effet prolifératif de cellules et leur isolément

(30) Priority: 10.06.1988 JP 143221/88
(43) Date of publication of application: 13.12.1989
(73) Proprietor: SANWA KAGAKU KENKYUSHO CO., LTD., Higashi-ku Nagoya-shi Aichi-ken (JP)
(72) Inventor: Seyama, Yousuke, Bunkyo-ku Tokyo (JP); Kano, Kazutaka, Mitaka-shi Tokyo (JP); Yokoyama, Yoshiko, Bunkyo-ku Tokyo (JP); Kaji, Kazuhiko, Omiya-shi Saitama-ken (JP); Sawai, Kiichi, Funabashi-shi Chiba-ken (JP)
(74) Representative: Diamond, Bryan Clive

(56) References cited:
- WO-A-81/00260
- CHEMICAL ABSTRACTS, vol. 93, no. 9, 1st September 1980, page 388, abstract no.92388d, Columbus, Ohiom, US; M. YOSHIMURA et al.: "Mucosubstances of the aciniof the rabbit harderian gland: a histochemical and electron microscopic study",& NIPPON GANKA GAKKAI ZASSHI 1980, 84(2), 101-12

## Description

The present invention relates to a protein capable of promoting the proliferation of cells (hereinafter referred to as the cell-proliferative protein), which is present in Harder's glands of mammalian animals, and a process for the isolation thereof.

In recent years, various augmentors (or cell-proliferation augmenting factors) have attracted a good deal of attention and been studied, leading to the discovery of various augmenting factors originating from animal cells, human cells and bacteria. These factors have now been mass-produced by the use of genetic engineering.

As the factors originating from animal cells, mouse EGF JP-A-57-206617), placental growth factors (JP-A-61-229894 and prostatic growth factors (JP-A-62-181222, have been found.

The application of such factors to wound and ulcer remedies have been investigated, but they are still on their way to research and development. In most cases, they have thus been disadvantagous in that they are unsuitable for actual treatments due to their poor stability, have specific selectivity in view of their action, and are of strong toxicity. In addition, they are often difficult to produce industrially.

An object of the present invention is to provide a cell-proliferative protein free from such disadvantages as mentioned above and a process for the isolation thereof.

According to the present invention, the above object is achieved by the provision of a cell-proliferative protein which is present in Harder's glands of mammalian animals.

The protein according to the present invention should have the following physicochemical properties.
(A) The molecular weight is in a range of 14,000 to 15,000 as measured by SDS polyacrylamide electrophoresis.
(B) The isoelectric point is in a range of 4.5 to 5.5, and
(C) The number of residues relative to one methionine residue is 2.8 for asparagine and asparaginic acid, 1.6 for glutamine and glutamic acid, 1.0 for methionine, 2.5 for alanine, 3.5 for isoleucine, 13.3 for serine, 3.2 for leucine, 11.8 for lysine and 2.4 for valine.

The protein according to the present invention may be isolated by chromatographing a degreased liquid extract of Harder's glands of a mammalian animal, using an ion exchange resin, a dye bonded resin and a molecular sieve resin.

The thus isolated protein may be formed into stable compositions, which may then be provided as pharmaceutical preparations for treating human and animal diseases such as various diseases due to cytoclasis, for instance, hepatic diseases, ulcers and wounds.

In order to make various preparations, the cell-proliferative protein according to the present invention may be used with protein stabilizers such as glycerols and albumins for stabilizing purposes, since the present protein is very unstable. As the glycerols, use may be made of, e.g., glycerin and polyethylene glycol.

With the cell-proliferative protein according to the present invention, it is possible to subculture various useful cells derived from human beings and various animals and culture them in a culture medium containing no serum. These properties make the present protein especially effective for the preparation of artificial skins or livers as well as collagen.

When the present protein is used as pharmaceutical material , it may be specifically effective for various cytoclastic diseases, for instance, hepatic diseases, ulcers and dermal diseases (chapping, burns, frostbite, chilblains, etc.). In addition, the present protein may be used as a pre-treating material for tissue activation in skin grafting.

Pharmaceutical compositions containing the cell-proliferative protein according to the present invention may be stabilized for immediate use. Alternatively, they may be powdered or granulated with suitable carriers or excipients to prepare powders, granules, tablets, etc., or may be available in a form for external application.

Preferably, a daily dosage of the present protein to adults is in a range of about 10 ng to 100 mg.

In the accompanying drawings:
Figure 1 illustrates an elution pattern of a TSK DEAE-5PW column in the process of purification with ● denoting the quantity of protein and ○ standing for the trapping quantity of ³H-thymidine in terms of radioactivity,
Figure 2 illustrates an elution pattern of Blue Cephalose CL6B in the process of purification,
Figure 3 illustrates an elution pattern of a Superlose 12 column in the process of purification,
Figure 4 illustrates a pattern of isoelectrophoresis, and
Figure 5 illustrates the results of the proliferative activity on cells K2T1 and TIG-3, as measured.

### EXAMPLES

The present invention will now be explained in further detail with reference to the following test and preparation examples.

### Examples - Separation, Purification, etc. of Cell-Proliferative Protein

### (1) Determination of Cell-Proliferative Activity

Pulmonary fibroblasts (TIG-3) of a human embryo are removed from a Schale dish by treatment with trypsin, and are thereafter inoculated into an MCBD-104 culture medium available from GIBCO (U.S.A.) at a concentration of 7.5 × 10⁴ cells/ml, said culture medium being freed of zinc but containing swine insulin (5 µg/ml), human transferrin (1 µg/ml) and dexamethasone (10 ng/ml). Then, 80 µl portions of the culture medium are poured into a 96-hole plate. In this state, the cells are incubated at 37°C in an incubator containing 5 % carbon dioxide for 2 or 3 days until they are put into a confluent state suitable for the determination of activity. Ten (10) µl portions of ³H-thymidine (91 Ci/mmol, available from Amersham) and a sample dissolved in 20 mM Tris-HCl containing 0. 14 M NaCl, pH 7.8, are added to the culture medium for further 48-hour incubation at 37°C.

The cells are washed with HBS-thymidine (10 mM HEPES containing 0.14 M NaCl, 10 mM KCl, 1 mM EDTA and 1 mM thymidine, pH 7.2), and are then incubated at 37°C for 10 minutes with the addition of 100 µl of trypsin (20U). Afterwards, the cells are trapped in a GF/C filter, available from Whatman, with the use of a semi-automatic cell harverster. The radioactivity of the cells trapped in this filter is measured. The sample was diluted with solutions of 20 mM Tris-HCl, pH 7,8, containing 5 % glycerin and 5 mg/ml of BSA.

### (2) Separation and Purification of Cell-Proliferative Protein

After chopping, 70 g of Harder's glands obtained from 140 guinea pigs are homogenized in a HEPES buffer containing 0.15 M NaCl and 5 % glycerin, pH 7.2 (hereinafter abbreviated as TGB). After tissue residues are removed by centrifugation at 20,000 ×g for 1 hour, the glands are sterilized through a Millex HA filter (Millipore Co., Ltd.). The thus obtained crude liquid extract (equivalent to 730 mg of protein) is washed with TGB in a 2.1 × 15 cm column of TSK DEAE-5PW (an ion exchange resin) pre-equilibrated with TGB.

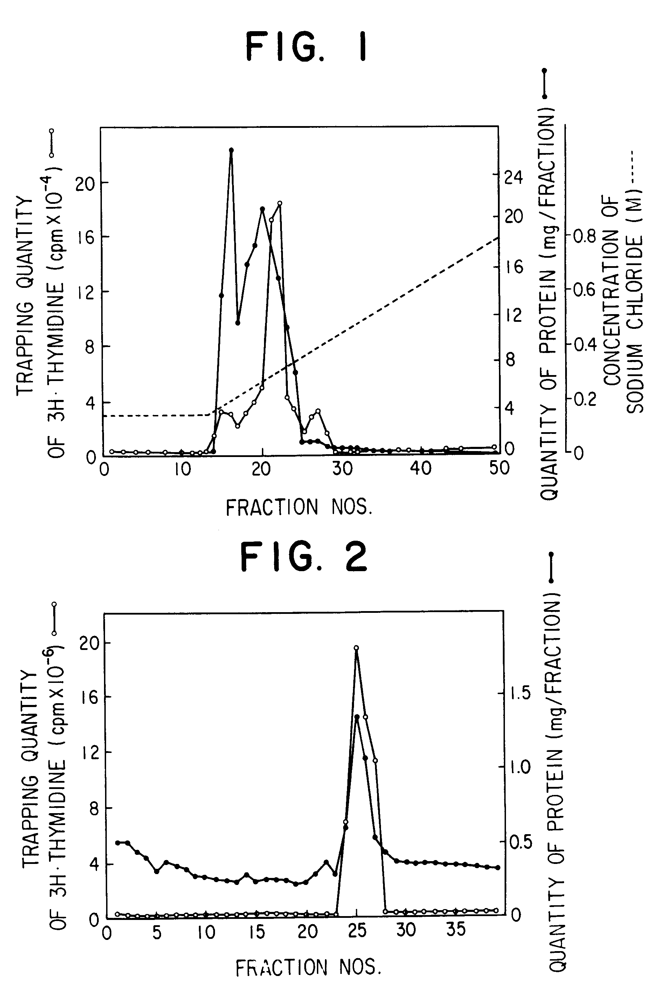

Fractions eluted from this column at a linear gradient of 0.15 to 0.80 M NaCl were collected in 3 ml proportions, and were measured in terms of the quantity of protein with a protein measuring kit, available from Bio-Rad, and the cell-proliferative activity with the procedures described in (1). The results are plotted in Figure 1.

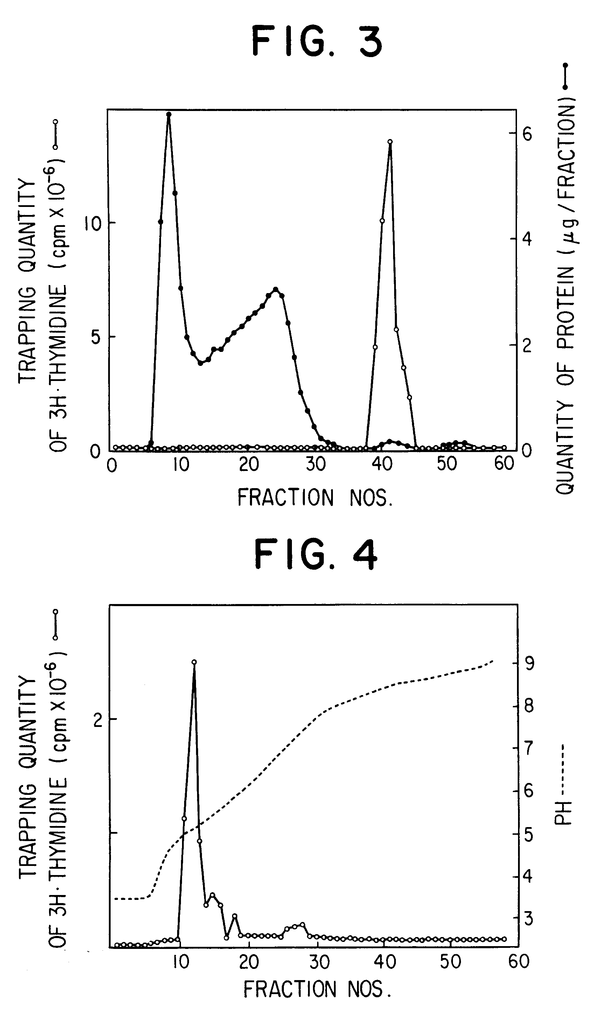

Referring to the cell-proliferative activity, elution is found at positions of 0.2 to 0.35 M NaCl. The fractions are then collected and treated through a 2.4 × 24 cm column of Blue Cephalose CL-6B (a dye-bonded resin) pre-equilibrated with TGB. The column was washed with TGB containing 0.2 M NaCl and then with TGB containing 250 ml of 0.6 M NaCl. Subsequent elution with TGB 2.2 M NaCl gave 4 ml proportions of fractions, which were then measured in terms of the quantity of protein and the cell-proliferative activity, as mentioned above. The obtained chromatopattern is shown in Figure 2. Referring to the cell-proliferative activity, elution was found at Fraction Nos. 24 to 27. The active fractions were further subjected to gel filtration at a flow rate of 1 ml/minute through an 1.6 × 50 cm column of Superlose 12 containing 1.5 M NaCl (a molecular sieves resin) to obtain 1 ml portions of filtrates, which were then measured in terms of the quantity of protein and the cell-proliferative activity, as mentioned above. The obtained chromatogram is shown in Figure 3.

In this case, the cell-proliferative activity was found to be eluted at Fraction Nos. 40 and 41. In this stage, 18 % of SDS-PAGE (polyacrylamide electrophoresis) indicated that a cell-proliferative protein was detected in the form of a single band at positions expressed by a molecular weight of 14000 to 15000. Thus, the purification of protein was completed.

Yields, etc. in the respective steps of purification are summarized in Table 1. With this purification process, it was possible to purify 5.6 µg of the cell-proliferative protein from Harder's gland protein in an about 10 % yield.

**Table 1**

| | Quantity of Protein (mg) | Rising Factor of Specific Activity | Yield (%) |
|---|---|---|---|
| Crude Liquid Extract | 2190 | 1 | 100 |
| Ion Exchange Resin | 279 | 7.7 | 76 |
| Blue Cephalose | 2.12 | 226 | 20 |
| Superlose 12 | 0.0056 | 43080 | 10 |

### (3) Measurement of Isoelectric Point

Under such conditions as expressed in terms of 500 V, 48 hours and 4°C, the isoelectric focusing of 200 µg of an active fraction of Blue Cephalose GL-6B was carried out in a solution containing 1 % of anphorite (LKB Co., Ltd.) at a sucrose density gradient through 8101 Column, avaialble from LKB to find an isoelectric point peak at which the greatest cell-proliferating activity was obtained. The results are plotted in Figure 4, from which the isoelectric point is found to be 4.5 to 5.5.

### (4) Compositional Analysis of Amino Acids

One (1) mg of an active fraction of Superlose 12 dissolved in 1 ml of 6 N HCl was frozen with dry ice-acetone, followed by sufficient degasification. This was sealed in a tube and acid-hydrolyzed at 110±1°C for 24 hours. After cooled down to room temperature, the tube was unsealed. The content was dried into solid under reduced pressure in a rotary evaporator, and was then kept stationary at room temperature with the addition of 0.5 ml of a solution of 0.01 N NaOH. Additionally, 1.5 ml of 0.02 N hydrochloric acid was added. Fifty (50) µl of the product were measured with a fast amino acid analyzer, Type 835 available from Hitachi. As a result, the measurements summarized in Table 2 was obtained.

**Table 2**

| Composition of Amino Acids | Number of Residues (relative to one methionine residue) |
|---|---|
| Methionine | 1.0 |
| Asparagine and Asparaginic Acid | 2.8 |
| Glutamine and Glutamic Acid | 1.6 |
| Glycine | 9.1 |
| Alanine | 2.5 |
| Isoleucine | 3.5 |
| Serine | 13.3 |
| Leucine | 3.2 |
| Lysine | 11.8 |
| Valine | 2.4 |
| Note that phenylalanine, tryptophan, threonine, histidine, arginine and tyrosine were unmeasurable. | |

### Test Examples

### 1: Measurement of Cell-Proliferative Activity

### (1) Proliferative Activity upon Fibroblasts

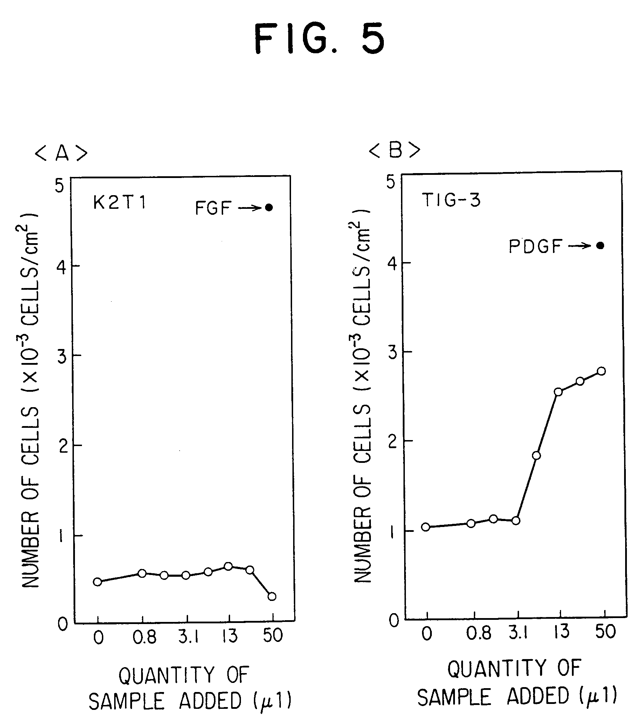

For the purpose of comparison with acidic FGF of the cell-proliferative factor already known in the art, cells K2T1 sensitive to FGF and derived from a human umbilical vein were incubated at 37°C in an MCBD-104 culture medium containing 10 % of a blood serum of a bovine embryo. The results are given in Figure 5, wherein ○ stands for the number of cells incubated in the culture medium when 0.8 to 50 µg of a fraction (150 µg/ml) of Blue Cephalose containing the substance of the present invention were added thereto, and ● denotes the number of cells incubated in the same culture medium when 70 µg of acidic FGF, 10 ng/ml of EGF and 100 µg of heparin were added thereto.

In Figure 5(B), ○ stands for the number of cells obtained by incubating fibroblasts TIG-3 derived from the lungs of a human embryo in an MCBD-104 culture medium containing 5 µg/ml of insulin, 5 µg/ml of transferrin and 100 µg/ml of heparin with the addition of 0.8 to 50 µl of a fraction (150 µg/ml) of Blue Cephalose. From these results, it has been found that the substance of the present invention has no substantial cell-proliferative activity upon the cells K2T1 sensitive to FCF, but shows increased cell-proliferative activity upon the cells TIG-3. Additionally, it has been found that the present substance has an increased cell-proliferative effect upon cells derived from the cornea of a rabbit and the skin of a horse.

### (2) Proliferative Effect on the Hepatocellular Cultivation

Hepatocytes were separated and prepared from Wistar rats (weighing 180 to 200 g) by the continuous collagenase process (Tanaka, K, et al., "Biochem.", 84, 937 to 946, 1978).

According to the process of Nakamura et al (Japanese Patent Laid-Publication No. 60-45534), the cell-proliferative effect was measured in terms of the trapping activity of ³H-thymidine into DNA fractions (dpm/mg prtein/hr). The results are summarized in Table 3.

**Table 3**

| Proliferative Factor | Concentration | DNA Synthesis (dpm/mg protein/hr) |
|---|---|---|
| No factor added | | 500 |
| EFG | 20 ng/ml | 11500 |
| Present Substance | 10 ng/ml | 18400 |

As will be understood from the above results, the present substance is much superior in the proliferative effect to EGF heretofore known to be a proliferative factor upon hepatocytes.

### (3) Curative Effect on Wounds Induced by Experimental Incision

The back region of a male SD rat (of 9-week age) was shaved off, sterilized with 70 % alcohol, and incised by a knife over a length of 1 cm along the median line under anesthesia to induce an experimental wound. The test compound was then applied over that wound to visually observe its state after 3, 6 and 10 days of the commencement of experimentation. It is noted that the test compound was prepared according to Preparation Example 4 to be described later.

The results of observation of the depth of the wounds are summarized in Table 4.

**Table 4**

| Groups | Grade | 3 days | 6 days | 10 days |
|---|---|---|---|---|
| Control Group | - | 10 | 9 | 0 |
| | + | 0 | 1 | 7 |
| | ++ | 0 | 0 | 3 |
| Test Group | - | 9 | 8 | 0 |
| | + | 1 | 2 | 3 |
| | ++ | 0 | 0 | 7 |
| The grades used for the visual observation of the curative process of wounds are: ++ = shallow, + = medium and - = deep. | | | | |

In each group, 10 rats were used.

In the control group, only three wounds were found to be shallow. In the test group, however, as many as 7 wounds were found to be shallow. Thus, the present preparation has a curative effect on wounds.

### 2: Pharmaceutical and Pharmacological Effect Tests

One (1) mg/kg of carbon tetrachloride was administrated to two groups, each of five male rats weighing about 200 g, twice a week over ten weeks to induce hepatic diseases. A preparation according to Preparation Example 1 was continuously and hypodermically administrated to the test group at a daily dosage of 100 µg/kg over 14 days, while physiological saline was simiarly administered to the control group. The blood was optionally gathered from the caudal veins to measure GOT and GPT. As a result, the curing factor was increased 40 % or more.

### 3: Use Example

After shaving and washing, ten (10) male panelists were made to use a cream preparation according to Preparation Example 4 over two weeks when they were questioned about its effects, with the following results:

| (1) The Touch | Number of Answers |
|---|---|
| Good | 8 |
| Bad | 0 |
| Indistinguishable | 2 |

| (2) Amelioration of Chapping and Razor Rash | Number of Answers |
|---|---|
| Effective | 8 |
| Indistinguishable | 2 |
| Adversely Affected | 0 |

### 3: Preparation Examples

| Preparation Example 1 (injection) | |
|---|---|
| Substance of the Invention | 150 µg |
| Sodium acetate | 3 mg |
| P-oxymethyl benzoate | 2 mg |
| Glycerol | 30 mg |
| Polysorbate | 30 µg |
| Human albumin | 5 mg |
| Distilled water for injection | suitable quantity |
| Sodium hydroxide | suitable quantity |
| Regulated to pH 7.4. | |

The above components were mixed together in the specified proportion, and the mixture was sealed in ampules through a sterilized membranous filter in 2 ml portions.

| Preparation Example 2 (Dental Paste) | |
|---|---|
| Insoluble sodium methaphosphate | 40.0 % |
| Glycerin | 10.0 % |
| Sorbitol | 10.0 % |
| Carboxymethyl cellulose | 1.0 % |
| Sodium lauryl sulfate | 2.0 % |
| Sodium saccharate | 0.5 % |
| Perfumes | 0.5 % |
| Substance of the invention | 1 ppm |
| Water | Balance |

From the above composition, dental paste was prepared in conventional manner.

| Preparation Example 3 (Cough Preparation) | |
|---|---|
| Ethyl alcohol | 10.0 % |
| Sodium saccharate | 0.1 % |
| Perfumes | 1.0 % |
| Polyoxyethylene sorbitan laurate | 1.0 % |
| Substance of the invention | 0.0001 ppm |
| Water | Balance |

| Preparation Example 4 (O/W Cream) | |
|---|---|
| Substance of the invention | 0.01 % |
| Liquid paraffin | 10.0 % |
| Butylene glycol | 5.0 % |
| Beeswax | 2.0 % |
| Cetyl alcohol | 4.0 % |
| Lanolin | 2.0 % |
| Squalane | 30.0 % |
| Paraben | 0.2 % |
| Polyoxyethylene monosorbitan laurate | 2.0 % |
| Refined water | Balance |

| Preparation Example 5 (Rouge - Lip Preparation) | |
|---|---|
| Substance of the invention | 0.005 % |
| Paraffin | 2.0 % |
| Castor oil | 55.0 % |
| Lanolin | 11.0 % |
| Isopropyl myristate | 10.0 % |
| Titanium oxide | 2.0 % |
| Red No. 202 | 3.0 % |
| Perfumes | 0.1 % |
| Waxes | Balance |

## Claims

1. A cell-proliferative protein present in Harder's glands of mammalian animals and having the following physicochemical properties:
(A) the molecular weight being in a range of 14,000 to 15,000 as measured by SDS polyacrylamide electrophoresis,
(B) the isoelectric point being in a range of 4.5 to 5.5, and
(C) the number of residues relative to one methionine residue being 2.8 for asparagine and asparaginic acid, 1.6 for glutamine and glutamic acid, 1.0 for methionine, 2.5 for alanine, 3.5 for isoleucine, 13.3 for serine, 3.2 for leucine, 11.8 for lysine and 2.4 for valine.

2. A cell- proliferative protein as claimed in Claim 1, which is effective for the subculture of useful cells derived from human beings and various animals and the culture thereof in a culture medium containing no serum.

3. A cell- proliferative protein as claimed in Claim 1 or 2, which has a strong proliferative effect upon hepatocytes.

4. A process for isolating a protein as claimed in Claim 1, 2 or 3 wherein a degreased liquid extract of Harder's glands of a mammalian animal is isolated by chromatography using an ion exchange resin, a dye bonded resin and a molecular sieve resin.

5. A pharmaceutical composition containing a cell-proliferative protein as claimed in any of Claims 1 to 3.

## Patentansprüche

1. Zellwachstumsförderndes Protein, das in Harder'schen Drüsen von Säugetieren vorhanden ist, und welches die folgenden physikalisch-chemischen Eigenschaften hat:
(A) das Molekulargewicht ist im Bereich von 14'000 bis 15'000 bei der Messung mit der SDS Polyacrylamide-Elektrophorese,
(B) Der isoelektrische Punkt liegt im Bereich von 4.5 bis 5.5 und
(C) die Zahl der Residuen, bezogen auf ein Methionin Residuum ist 2.8 für Asparagin und aspartic acid, 1.6 für Glutamin und Glutaminsäure, 1.0 für Methionin, 2.5 für Alanin, 3.5 für Isoleucin, 13.3 für Serin, 3.2 für Leucin, 11.8 für Lysin und 2.4 für Valine.

2. Zellwachstumsförderndes Protein nach Anspruch 1, welches bei der Subkultur von Hilfszellen wirksam ist, die von Menschen und verschiedenen Tieren stammen und deren Kultur in einem Nährmedium, das kein Serum enthält.

3. Zellwachstumsfördernde Protein nach Anspruch 1 oder 2, welches einen stark wachstumsfördernden Einfluss auf Hepatocyten hat.

4. Verfahren zum gewinnen eines Proteins nach einem der Ansprüche 1, 2 oder 3, bei welchem ein entfettetes, flüssiges Extrakt von Harder'schen Drüsen von Säugetieren chromatographisch isoliert wird, bei der ein ionentauschendes Harz, ein farbstoffgebundenes Harz und ein Molekularsieb-Harz verwendet wird.

5. Ein pharmazeutisches Präparat welches ein zellwachstumsfördernde Protein nach einem der Ansprüche 1 bis 3 enthält.

## Revendications

1. Protéine pour la prolifération de cellules présente dans les glandes de Harder de mammifères, ayant les propriétés physico-chimiques suivantes :
(A) un poids moléculaire dans la gamme allant de 14.000 à 15.000, ce poids moléculaire étant mesuré par électrophorèse sur un gel de dodécyl sulfate de sodium et de polyacrylamide,
(B) un point isoélectrique dans la gamme allant de 4,5 à 5,5 et
(C) un nombre de groupes rapportés au groupe méthionine de 2,8 pour l'asparagine et l'acide aspartique, 1,6 pour la glutamine et l'acide glutamique, 1,0 pour la méthionine, 2,5 pour l'alanine, 3,5 pour l'isoleucine, 13,3 pour la sérine, 3,2 pour la leucine, 11,8 pour la lysine et 2,4 pour la valine.

2. Protéine pour la prolifération des cellules selon la revendication 1, qui est efficace pour la sous-culture de cellules utiles dérivées d'humains et de différents animaux et leur culture dans un milieu de culture ne contenant pas de sérum.

3. Protéine pour la prolifération des cellules selon la revendication 1 ou 2, qui a un effet très favorable sur la prolifération des hépatocytes.

4. Procédé pour isoler une protéine selon la revendication 1, 2 ou 3, dans lequel un extrait liquide dégraissé des glandes de Harder d'un mammifère est isolé par chromatographie en utilisant une résine échangeuse d'ions, une résine liée à un colorant et une résine constituant un tamis moléculaire.

5. Composition pharmaceutique contenant une protéine pour la prolifération de cellules selon une quelconque des revendications 1 à 3.
